# EUROPÄISCHE PATENTSCHRIFT

(11) **EP 0 950 377 B1**
(45) Veröffentlichungstag und Bekanntmachung des Hinweises auf die Patenterteilung: **27.07.2005**
(21) Anmeldenummer: 99250120.5
(22) Anmeldetag: 14.04.1999
(51) Int. Cl.: A61B 18/12

(54) **Ablationsanordnung**
Ablation Assembly
Dispositif d'ablation

(30) Priorität: 15.04.1998 DE 19817553
(43) Veröffentlichungstag der Anmeldung: 20.10.1999
(73) Patentinhaber: BIOTRONIK Mess- und Therapiegeräte GmbH & Co Ingenieurbüro Berlin, 12359 Berlin (DE)
(72) Erfinder: Wetzig, Thomas, 81825 München (DE); Graf, Markus, 12165 Berlin (DE); Sachse, Steffen, 02159 Dresden (DE); Pohl, Olaf, 01796 Pirna (DE)
(74) Vertreter: Eisenführ, Speiser & Partner

(56) Entgegenhaltungen:
- WO-A-96/00036
- US-A- 5 462 544
- US-A- 5 570 218
- US-A- 5 722 401

## Beschreibung

Die Erfindung betrifft eine Ablationsanordnung gemäß dem Oberbegriff des Anspruchs 1.

Derartige Ablationsanordnungen sind bekannt und in der klinischen Anwendung bewährt. Die Energiequelle (z.B. ein HF-Generator und -verstärker) kann durch von Hand zu betätigende Schalter mit verschiedenen Elektroden eines multipolaren Katheters verbunden werden, um ein vom Arzt gewünschtes Läsionsmuster zu erzielen.

Diese von Hand zu schaltenden Anordnungen sind sehr flexibel einsetzbar, aber umständlich zu bedienen.

Eine weitere Ablationsanordnung ist aus der internationalen Patentanmeldung WO-A-97/20510 bekannt. Dort ist ein HF-Ablationssystem beschrieben, mit dem simultan und in vorgegebener Phasenzuordnung über mehrere Elektroden eines multipolaren Katheters HF-Energie abgegeben werden kann. Hierbei wird eine der Elektrodenzahl entsprechende Anzahl von Leistungs-Modulen eingesetzt.

Ein Mapping- und Ablationskatheter, der es dem Arzt erlaubt, zunächst intrakardiale Signale zum Zwecke der Diagnose über den Katheter aufzunehmen und anschließend eine Ablationsbehandlung mit Hilfe des Katheters durchzuführen ist aus dem US-Patent 5,722,401 bekannt. Außerdem ist aus der internationalen Patentanmeldung WO 96/00036 ein multipolarer Ablationskatheter bekannt, der Temperatursensoren zur Steuerung des Ablationsverlaufs aufweist.

Die letztgenannte Anordnung ist konstruktiv aufwendig und teuer. Außerdem sind die verschiedenen Elektrodensysteme nicht gegeneinander entkoppelt, so daß sich darauf basierende Meß- oder Regelkreise gegenseitig beeinflussen.

Der Erfindung liegt deshalb die Aufgabe zugrunde, eine leicht zu bedienende und kostengünstige Ablationsanordnung anzugeben.

Die Aufgabe wird durch eine Ablationsanordnung mit den im Anspruch 1 angegebenen Merkmalen gelöst.

Die Erfindung schließt den Gedanken ein, die Ablation insoweit zu automatisieren, als daß ein Modus der Zuschaltung der Energiequelle zu den einzelnen Auskoppelelementen zum Eintrag der Energie an vorbestimmten Punkten des Gewebes vorab im einzelnen bestimmbar ist und dann in einem automatischen Ablauf schnell und zeitgerecht ausführbar ist. Dies entlastet den Arzt von der Aufgabe, während es Eingriffs ständig Schalter zu betätigen, ohne ihn in der Freiheit der Positionierung der Läsionsbereiche entsprechend dem konkreten Befund einzuschränken. Im Gegenteil kann er seine Aufmerksamkeit verstärkt dem Patienten bzw. der Katheterpositionierung widmen. Durch die Verkürzung der Ablationsprozedur sinken die Risiken der Strahlenbelastung und der Katheterfehlpositionierung.

Gemäß der Erfindung sind hierzu ein Ablationsprogrammspeicher mit einer der Anzahl der Energieauskoppelelemente entsprechenden Anzahl belegter Speicherbereiche sowie Mittel zur Beeinflussung eines im Ablationsprogrammspeicher gespeicherten Ablationsprogramms in Abhängigkeit von mindestens einer vor oder während der Behandlung eingegebenen und/oder einer während der Behandlung gemessenen Größe vorgesehen. Während also das grundsätzliche Regime der sequentiellen Zuschaltung der einzelnen Auskoppelpunkte mit der Energiequelle vorab festgelegt wird, bestehen - neben den außerdem vorhandenen Möglichkeiten für eine Notabschaltung - Freiheitsgrade vor allem hinsichtlich des konkreten zeitlichen Ablaufes und insbesondere der Berücksichtigung von aktuell gewonnenen Meßwerten, die den Stand der Behandlung reflektieren.

In einer der bevorzugten Ausführungen als HF-Ablator sind die Energiequelle als HF-Spannungsquelle (mit entsprechendem Verstärker), das Halteelement als Katheterkörper, die Energieauskoppelelemente als Elektroden, die Energieübertragungsleitungen als elektrische Leitungen (diese Teile zusammen mithin als an sich bekannter multipolarer Ablationskatheter) und die Schaltelemente als elektromechanische oder elektronische Schalter ausgebildet.

Sowohl die Energiequelle als auch verschiedenste mit der Erfindung einsetzbare Ablationskatheter sind als solche bekannt und brauchen hier nicht näher beschrieben zu werden. Als Schaltelemente können z.B. Relais oder Halbleiterschalter (bevorzugt in CMOS-Technik) eingesetzt werden. Die Erfindung ermöglicht eine wesentliche gerätetechnische Vereinfachung und Kostensenkung, indem nur eine einzelne Energiequelle - speziell ein einziger HF-Verstärker - im Multiplexbetrieb vorgesehen ist. Durch die Möglichkeit synchroner Umschaltungen der HF-Leitung und der Temperaturmeßstellen können mehrere Temperaturregelkreise ohne gegenseitige Beeinflussung betrieben werden.

Grundsätzlich ist die Erfindung auch bei andersartigen Ablatoren einsetzbar - wenn etwa die Energiequelle als Hochleistungs-Strahlungsquelle, insbesondere Laserquelle, das Halteelement als Katheterkörper, die Energieübertragungsleitungen und Energieauskoppelelemente als Lichtwellenleiteranordnung und die Schaltelemente als elektrooptische Schaltelemente ausgebildet sind.

Die Energieauskoppelelemente dienen in vorteilhaften Ausführungen zugleich als Meßfühler, und es ist eine den Energieübertragungsleitungen zuschaltbare Meßsignal-Verarbeitungseinheit zur Auswertung der aufgenommenen Meßsignale vorgesehen, wobei die einzelnen Energieauskoppelelemente unter Steuerung durch die Programmsteuermittel über Schaltelemente dem Eingang der ersten Meßsignal-Verarbeitungseinheit zugeschaltet werden.

Speziell beim HF-Ablator sind die Elektroden zur Aufnahme von monophasischen Aktionspotentialen (MAP) und die erste Meßsignal-Verarbeitungseinheit zu deren Auswertung ausgebildet. Bei einem Gerät zur Läsion von Herzgewebe zur Behandlung von Tachyarrhythmien ist zudem ein Stimulationsimpulsgenerator vorgesehen, der mit den Energieübertragungsleitungen und somit unter Steuerung durch die Programmsteuermittel über die Schaltelemente mit den Elektroden verbindbar ist. Bei einem Laserablator können über die Glasfaseroptiken zur Einkopplung der Laserenergie in das Gewebe grundsätzlich zugleich zur Beobachtung des Gewebes bzw. zur optischen Meßwerterfassung genutzt werden.

Mittels der Programmsteuerung wird bei diesen Ausführungen nicht nur der Therapieablauf, sondern auch die zugehörige Meßwerterfassung und gegebenenfalls vorangehende Stimulation vereinfacht, was die Bedienung für den Arzt weiter erleichtert.

Die oben erwähnten Mittel zur Beeinflussung des Ablationsprogramms umfassen insbesondere eine Mehrzahl von zusätzlichen (jeweils den Energieauskoppelementen zugeordneten) Meßfühlern, insbesondere Temperaturfühlern, eine an diese angepaßte Meßsignal-Verarbeitungseinheit und eine mit deren Ausgang verbundene Speicherzugriffssteuerung zur Variation der Rate und/oder Reihenfolge des Zugriffs zu den Speicherbereichen des Ablationsprogrammspeichers in Abhängigkeit von den Meßsignalen bzw. dem Auswertungsergebnis.

Den Meßfühlern ist zur weiteren Vereinfachung der Anordnung ein durch die Programmsteuermittel in Abhängigkeit vom Ablationsprogramm gesteuerter Multiplexer zur Übertragung der Meßfühlersignale über eine gegenüber der Anzahl der Meßfühler verringerte Anzahl von Meßsignal-Übertragungsleitungen zugeordnet. Die an eine programmgesteuert arbeitende Anordnung der oben skizzierten Art zu stellenden Sicherheitsanforderungen werden dadurch erfüllt, daß die Programmsteuermittel mindestens einen - bevorzugt zwei - Abschalt-Zeitgeber zur Sicherheitsabschaltung aller Energieübertragungsleitungen bei Erfüllung eines oder mehrerer vorbestimmter Abschaltkriterien aufweisen.

Indem bei einem vorteilhaften Ausführungsbeispiel der Erfindung die Programmsteuermittel und die Schaltelemente in einem separaten Schaltgerät untergebracht sind, das eingangsseitig über zwei serielle Datenleitungen, eine Stromversorgungsleitung und einen ersten Abschnitt der Energieübertragungsleitung mit einem herkömmlichen Ablationsgerät verbunden ist, kann aus vorhandenen Ablationsgeräten und Kathetern unter Hinzunahme von erfindungsgemäß ausgebildeten Komponenten durch Nachrüstung mit geringem Zusatzaufwand eine moderne Anordnung mit hohem Gebrauchswert konfiguriert werden. In dieser Weise ist auch ein stufenweiser Ausbau neuer Geräte möglich. Insgesamt wird jedoch die integrierte Ausführung in einem Gerät zu bevorzugen sein.

Vorteilhafte Weiterbildungen der Erfindung sind im übrigen in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1 ein Blockschaltbild einer Ablationsanordnung gemäß einer bevorzugten Ausführung der Erfindung,
- Figur 2 als Detaildarstellung zu Fig. 1 ein Flußdiagramm zur Funktionsdarstellung einer softwaregesteuerten Programmsteuereinheit sowie
- Figur 3 als Ausführungsvariante ein Funktions-Blockschaltbild wesentlicher Komponenten einer anderen Programmsteuereinheit gemäß Figur 1 in hardwarenaher Darstellung.

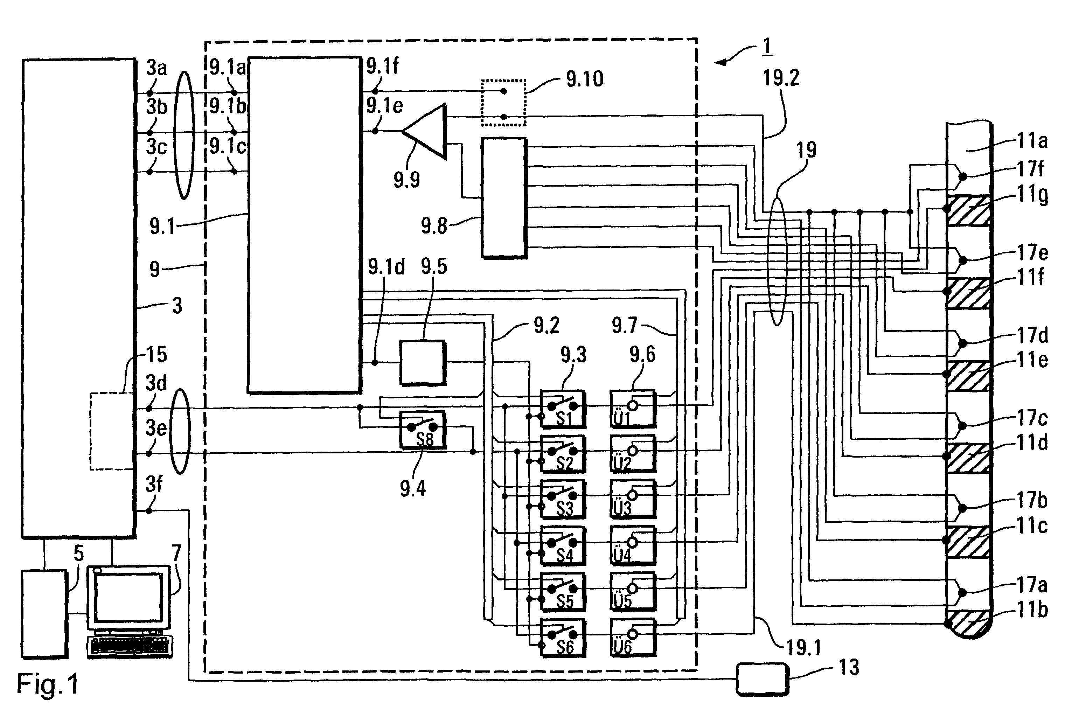

Die in Fig. 1 dargestellte Ablationsanordnung 1 besteht aus den Komponenten Ablationsgerät 3, Stimulationsgerät 5, klinischer Meßplatz 7, Schaltgerät 9, multipolarer Ablationskatheter 11 und Flächenelektrode 13, welche in bekannter Weise zusammenwirken. Für die Erläuterung der Erfindung sind dabei das Ablationsgerät 3, der HF-Generator 15 und die Ausgangsanschlüsse von besonderem Interesse. Dies sind zwei serielle Datenanschlüsse 3a, 3b, ein Stromversorgungsanschluß 3c, ein HF-Leistungsausgang 3d, 3e und ein Referenzanschluß 3f für die Flächenelektrode 13.

Auch der Ablationskatheter 11 ist von an sich bekannter Bauart und weist auf einem isolierenden, aus einem biokompatiblen Kunststoff gefertigten Katheterkörper 11a neben einer Spitzenelektrode 11b fünf äquidistant zueinander bzw. zur Spitzenelektrode angeordnete Ringelektroden 11c bis 11g auf, die - ebenso wie die Spitzenelektrode 11a - vorzugsweise aus Platin oder einer Pt-lr-Legierung gefertigt sind. Der Katheterkörper 11a nimmt (nicht einzeln bezeichnete) Zuleitungen für die einzelnen Elektroden sowie die - ebenfalls nicht einzeln bezeichneten - Konstantan- bzw. Kupferdrähte für den Elektroden räumlich zugeordnete Thermoelemente 17a bis 17f auf. In einer Verbindungsleitung 19 sind HF-Leitungen 19.1 und die Temperatursignalleitungen 19.2 vom Schaltgerät 9 zum Ablationskatheter 11 geführt.

Das Schaltgerät ist eingangsseitig (über wiederum nicht gesondert bezeichnete Leitungen) mit den Ausgangsanschlüssen 15a bis 15e des Ablationsgerätes verbunden. Es umfaßt als wichtigste Komponenten für die Ablationssteuerung einen Mikrocontroller 9.1, sechs mit dem Mikrocontroller über eine Busleitung 9.2 verbundene elektronische Schalter 9.3 für die Katheterelektroden 11b bis 11g sowie einen Sicherheitsausschalter 9.4 und schließlich einen Watchdog-Timer 9.5. Den HF-Ausgangsleitungen 19.1 sind Stromüberwachungsfühler 9.6 zugeordnet, die über eine zweite Busleitung 9.7 mit dem Mikrocontroller 9.1 verbunden sind. Der Mikrocontroller ist über Datenein- und -ausgänge 9.1a. 9.1b mit den Datenein- und -ausgängen 3a, 3b des Ablationsgerätes und über einen Stromversorgungs-Eingang 9.1c mit dessen Stromversorgungs-Ausgang 3c verbunden, und für den Watchdog-Timer 9.5 ist ein separater Steuerausgang 9.1d vorgesehen.

Die Cu-Leitungen der Thermoelemente 17a bis 17f sind über einen Multiplexer 9.8 und die Konstantan-Leitung direkt mit einem einzelnen Meßverstärker 9.9 verbunden, und dessen Ausgang ist mit einem ersten T-Signaleingang 9.1d des Mikrocontrollers verbunden. Eine als Referenz dienende Kaltlötstelle 9.10 mit (nicht dargestelltem) Thermistor zur T-Erfassung ist direkt mit einem zweiten T-Signaleingang 9.1e des Mikrocontrollers verbunden.

Die primären Steuerfunktionen für eine Ablationsbehandlung (Elektrodenauswahl und -ansteuerfolge, Dauer und Abstände der HF-lmpulse etc.) werden nach Auswertung der mit Hilfe des Meßplatzes 7 gewonnenen Ergebnisse der klinischen Untersuchung aufgrund einer entsprechenden Programmierung durch den Arzt in dem (in der Praxis auch als "Ablator" oder "Ablationssystem" bezeichneten) Ablationsgerät 3 ausgeführt. Steuerbefehle des Ablationsgerätes werden über die Datenausgänge 3a, 3b dem Mikrocontroller 9.1 zugeführt, der unter anderem die Steuerbefehle in Steuersignale für die Schalter 9.3 und den Multiplexer 9.9 umsetzt, die Schaltfunktionen mittels der Stromfühler 9.6 überwacht und die Temperatur an den einzelnen Elektroden 17a bis 17f berechnet.

Mit der dargestellten Schalteranordnung ist die Abgabe von HF-Energie mit jeder beliebigen Ringelektrodenkombination (einschließlich Kombinationen mit der Flächen- bzw. Neutralelektrode 13) sowie die Applikation von Stimulationsimpulsen und die Detektion des intrakardialen Elektrogramms IEGM an den jeweils zugeschalteten Elektroden möglich. Durch synchrone Steuerung des Multiplexers 9.8 kann zugleich die Temperatur an den Elektroden überwacht werden, der Multiplexer kann aber im Bedarfsfall auch asynchron zu den Schaltern gesteuert werden. Die Ergebnisse der T-Überwachung werden an das Ablationsgerät 3 zur Anzeige für den Arzt und zur etwaigen Modifikation des Ablationsprogramms übergeben, können aber auch intern im Mikrocontroller 9.1 zur Auslösung einer Elektrodenabschaltung bei unzulässig hohen T-Werten genutzt werden.

Die Schalter 9.3 und 9.4 werden bevorzugt als MOSFET-Schalter ausgeführt, können aber - sofern die prinzipbedingte Limitierung der Umschaltgeschwindigkeit hinnehmbar ist - insbesondere auch durch Relais gebildet sein. Die bidirektionale Datenübertragung zwischen Ablator 3 und Schaltgerät 9 oder eine entsprechende Programmierung des Ablators ermöglicht die kurzzeitige Austastung der HF bei jeder Elektrodenumschaltung zur Verringerung der Schalterbeanspruchung, was vor allem bei Einsatz von Relais vorteilhaft ist.

Bei einer weiteren - nicht dargestellten Ausführung der Erfindung können - ein oder mehrere IEGM- und/oder Stimulationskanäle zwischen den Schaltern 9.3 und den Überwachungselementen 9.6 ein - bzw. ausgekoppelt werden und somit zusätzlich unabhängig von den Schaltern 3.3 für den HF-Pfad behandelt werden.

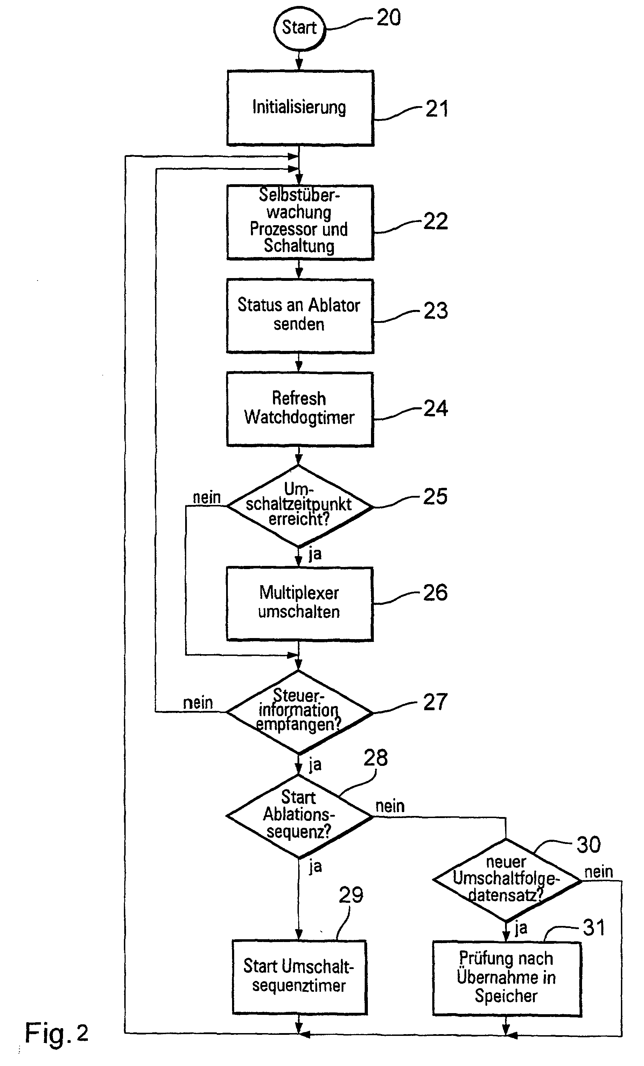

Bei dem in Figur 2 dargestellten Flußdiagramm ist in Form eines Programmablaufplans eine mögliche Ausführung eines Steuerprogramms für den Mikrokontroller 9.1 gemäß Figur 1 wiedergegeben.

Bei dem dargestellten Programmablauf des den Systemkontroller bildenden Mikrokontrollers ist zu erkennen, daß diese Software in Teile gegliedert ist, welche resident im Programmspeicher des Mikrokontrollers 9.1 gehalten sind, während andere Teile vor der Ablation wahlweise in den Mikrokontroller geladen werden können.

Die vom Mikrokontroller durchzuführenden Programmschritte ergeben sich dabei aus dem in Figur 2 dargestellten Ablauf: Nach dem Einschalten (Startmarke 20) führt der Mikrokontroller nacheinander eine Initialisierung (Block 21), einen Selbsttest (Block 22) und die Veranlassung der Einnahme eines Ausgangszustandes durch (Block 23). Der Programmspeicher befindet sich dabei zusammen mit dem Prozessor in dem Block 22.

Eine Ablationssequenz bildet dabei das "Ablationsprogramm" und besteht aus einer Folge von Umschaltsequenzen, deren Zeitfolge und Anzahl variabel ist. Während der Start der Ablationssequenz durch den Arzt durch die Abgabe eines Steuerbefehls, der die Ablationssequenz startet, bestimmt wird, ist - ausgehend von einem Grundprogramm - die Zeitfolge und Anzahl der Umschaltsequenzen variabel und ist aufgrund der vom Ablator empfangenen Signale veränderlich.

Die Umschaltzeitpunkte innerhalb der Sequenz werden durch den Watchdogtimer (Block 24) festgelegt. Wenn die Zeitdauer einer Ablationsperiode abgelaufen ist, (Raute 25) wird der Multiplexer (Block 26) umgeschaltet und die nächste Elektrode vom HF-Generator angesteuert. Im anderen Fall bleibt es bei dem bisherigen Schaltzustand. Falls inzwischen eine Steuerinformation empfangen wurde, welche den Programmablauf verändert (Temperatur- oder externe Steuerung) wird in der weiteren Raute 28 entschieden, ob die Steuerinformation eine neue Ablationssequenz einleitet. Andernfalls erfolgt ein Rücksprung hinter den Initalisierungsblock 21 und die Umschaltsequenz nach dem bisherigen Ablauf beginnt von neuem.

Im Falle des Empfangs einer Steuerinformation betreffend den Start einer neuen Ablationssequenz wird dies über den Block 29 durch ein entsprechendes Signal an den Block 22 veranlaßt. Wird bei der weiteren Raute 28 entschieden, daß es sich nicht um ein Startsignal handelt, wird vorausgestzt, daß ein neuer Umschaltfolgedatensatz vorliegt. Dies wird in der Raute 30 geprüft und im Falle einer erfolgreichen Überprüfung im Block 31 die Übernahme in den Speicher (Block 22) veranlaßt. Im anderen Fall wird der Speicher 22 mit dem unveränderten Signal beaufschlagt. Hierbei bearbeitet der Mikrokontroller also Programmteile zur Systemsicherung und wartet auf Steuerinformationen vom Ablator. Derartige Steuerinformation sind Datensätze mit Angaben zur Ringauswahl oder Informationen zum Starten oder Stoppen einer Ablationssequenz. Dabei setzt der Controller seriell vom Ablator empfangene Datensätze in Schaltinformationen um, welche das Ablationsprogramm ändern. Der Mikrokontroller ist in Figur 1 dabei mit dem Ablator 3 über die Anschlüsse 3a bis c verbunden.

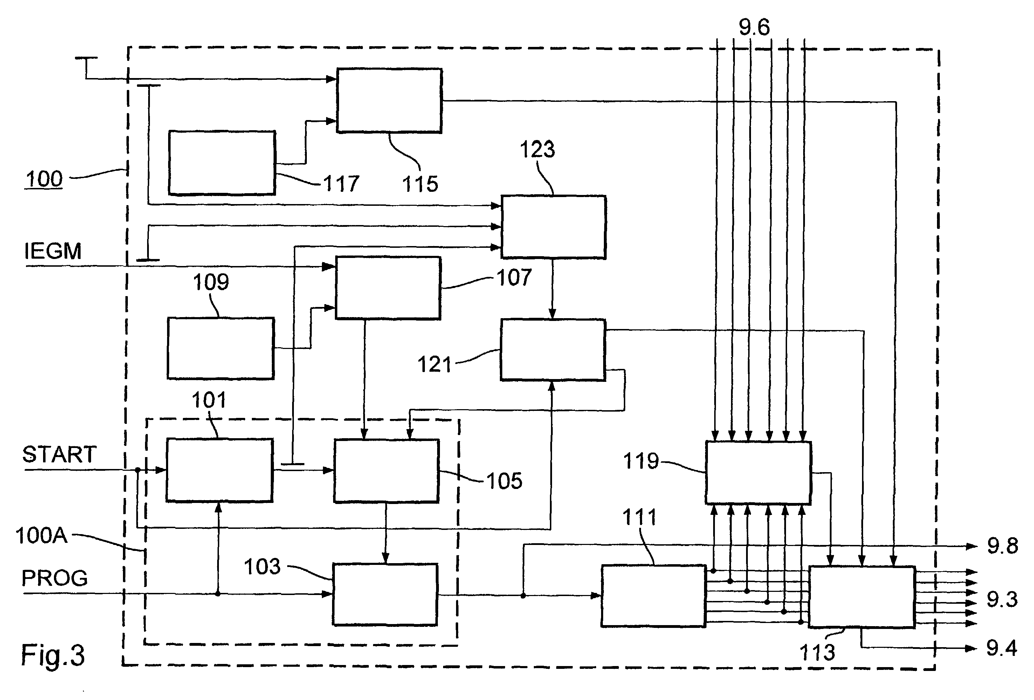

Eine weitere - hardwaremäßige Realisierung in Form eines Prinzip-Blockschaltbildes eine bevorzugte Ausführung wesentlicher Komponenten der Programmsteuereinheit 100 gemäß der Erfindung ist in Figur 3 dargestellt. Diese kann in der praktischen Realisierung aus Komponenten eines bekannten Ablationsgerätes und eines zusätzlichen Schaltgerätes gebildet sein (wie in Fig. 1 skizziert) oder eine Komponente eines neuartigen Ablationsgerätes darstellen.

Die Programmsteuereinheit 100 umfaßt als wesentliche Funktionsgruppe zunächst einen programmierbaren Zeitgeber 101, einen Schaltfolgespeicher 103 und eine diesem zugeordnete und eingangsseitig mit dem Zeitgeber 101 verbundene Speicherzugriffssteuerung 105, die zusammen einen Ablationsprogrammspeicher 100A bilden.

Die Speicherzugriffssteuerung 105 ist eingangsseitig weiterhin mit einer IEGM-Auswertungseinheit 107 verbunden, deren einem Eingang von einer (in der Figur nicht gezeigten) IEGM-Signalverarbeitungseinheit das jeweils aktuell über die aktive Elektrode des Ablationskatheters aufgenommene IEGM zugeführt wird und deren zweiter Eingang mit einem IEGM-Speicher 109 verbunden ist. Der Ausgang des Schaltfolgespeichers 103 ist hier mit einem Pufferspeicher bzw. Schaltstellungs-Halteglied 111 verbunden. An dessen Ausgang steht jeweils ein auf einen der Schalter 9.3 (Fig. 1) bezogenes Aktivierungssignal bereit, was in der Figur durch die Darstellung einer der Schalterzahl entsprechenden Anzahl von Ausgängen symbolisiert ist. Weiterhin ist der Ausgang des Schaltfolgespeichers mit dem den T-Fühlern 17a bis 17f zugeordneten Multiplexer 9.8 (Fig. 1) verbunden und aktiviert diesen entsprechend dem aktuellen Aktivierungssignal für die Elektroden.

Dem Schaltstellungs-Halteglied 111 ist ausgangsseitig ein Rücksetzglied 113 zugeordnet, welches seinerseits ausgangsseitig mit den Elektrodenschaltern 9.3 sowie zusätzlich dem HF-Zuleitungsschalter 9.4 verbunden ist. Das Rücksetzglied 111 ist an einem Steuereingang mit dem Ausgang einer Temperaturauswertungseinheit 115 verbunden, die ihrerseits über einen Eingang den Temperaturwert T an der aktuell angesteuerten Elektrode erhält und die über einen zweiten Eingang mit einem T-Vergleichswertspeicher 117 verbunden ist. Ein weiterer Steuereingang des Rüksetzgliedes 113 ist mit einer Schalterstrom-Auswertungseinheit 119 verbunden, die ihrerseits eingangsseitig mit den Stromüberwachungsfühlern 9.6 (Fig. 1) sowie mit dem Schaltstellungsspeicher 111 verbunden ist. Schließlich ist das Rücksetzglied über einen dritten Steuereingang, der mit den beiden anderen in ODER-Beziehung gesetzt ist, mit dem Ausgang eines Watchdog-Timers 121 verbunden. An diesen sind eingangsseitig über ein UND-Gatter 123 nicht gesondert bezeichnete Erfassungsmittel zur Erkennung einer regulären Übermittlung der IEGM- und T-Daten an die Programmsteuereinheit sowie von Timersignalen an die Speicherzugriffssteuerung 105 angeschlossen.

In Auswertung des klinischen Befundes und abgestimmt auf den konkret einzusetzenden Katheter wird vor jeder Behandlung eine - in der Figur durch das Signal " PROG" symbolisierte - Programmierung des Ablationsspeichers 100A, des Zeitgebers 101 und des Schaltfolgespeichers 103, vorgenommen. Mit dieser Programmierung wird ein Basis-Ablationsprogramm festgelegt, das die Schaltfolge und Einschaltdauer der einzelnen Ablationselektroden bestimmt.

Aufgrund eines "START"-Signals wird die Behandlung entsprechend diesem Programm eingeleitet, indem über den Zeitgeber 103 und die Speicherzugriffssteuerung 105 die erste Schalterstellung aus dem Schaltfolgespeicher 103 ausgelesen und im Pufferspeicher 111 festgehalten und zunächst der erste ausgewählte Schalter 9.3 aktiviert und über den Multiplexer 9.8 der zugehörige T-Fühler ausgewählt wird. Die ausgewählte Elektrode bleibt für die programmierte Zeit eingeschaltet, und anschließend wird die nächste im Ablationsprogramm vorgesehene Elektrode an den HF-Generator zugeschaltet, sofern nicht aufgrund eines besonderen Umstandes vom Arzt eine Modifikation des Basis-Programms veranlasst wird.

Eine solche Modifikation kann aufgrund einer Erfassung überhöhter Temperaturwerte am Katheter erfolgt durch eine über die Temperaturauswertungseinheit 115 gesteuerte Zurücksetzung des jeweiligen Schalters 9.3 über das Rücksetzglied 113. Weiterhin aktiviert der Watchdog-Timer 121 das Rücksetzglied und schaltet damit die gesamte HF bzw. die einzelnen Elektrodenzuleitungen ab, wenn Unregelmäßigkeiten in der Datenübertragung festgestellt werden. Schließlich erfolgt in analoger Weise, gesteuert über die Schalterstrom-Auswertungseinheit 119, eine Abschaltung, wenn auf einer anderen als der programmgemäß dem HF-Generator zugeschalteten Elektrodenzuleitung ein Strom erfaßt wird.

Eine Verzögerung der Schaltsequenz wird durch die IEGM-Auswertungseinheit 107 über die Speicherzugriffssteuerung 105 bewirkt, falls das während der Ablation erfaßte lokale Elektrogramm am Ort der aktivierten Elektrode bei Ablauf der programmierten Einschaltzeit nicht einem vorgegebenen Vergleichsmuster entspricht, was ein Indiz für eine noch nicht gelungene Gewebsläsion darstellt. Die für einen solchen Steuervorgang erforderlichen Auswertungen sind dabei relativ komplex.

In einer vereinfachten Variante - nicht näher dargestellten Variante - kann anstelle der Baugruppen 107 und 109 auch eine Eingriffsmöglichkeit in Form entsprechender Betätigungselemente für den Arzt vorgesehen sein, der dann eine Verlängerung der HF-Einwirkungsdauer gegenüber dem Basis-Ablationsprogramm aufgrund einer Beobachtung des IEGM-Signals durch eine manuelle Umschaltung veranlaßt. Hierzu wird über entsprechende Eingabemittel in den Speicher ein anderer geeigneter Programmablauf festgehalten. Ein Austausch der Programmdaten kann dabei bevorzugt durch ein Einlesen entsprechender Software von einem Datenträger erfolgen. Bei einer bevorzugten Ausführung sind die erfindungsgemäßen Programmsteuermittel in einem PC oder einem Laptop vorgesehen.

Die Erfindung beschränkt sich auch im übrigen in ihrer Ausführung nicht auf die vorstehend angegebenen bevorzugten Ausführungsbeispiele. Vielmehr ist eine Anzahl von Varianten möglich, welche von der dargestellten Lösung auch in anders gearteter Ausführung Gebrauch machen.

So können zur Temperaturerfassung im Ablationskatheter anstelle der oben erwähnten Thermoelemente auch Thermistoren vorgesehen sein, und deren Anzahl muß nicht gleich der Elektrodenzahl sein. Weiterhin kann nebend der Regstrierung überhöhter Temperaturen auch die Erfassung zu niedriger Temperaturen als Auslöser für Steuerfunktionen, insbesondere für eine Verlängerung der Elektroden-Einschaltzeit, dienen. In einer einfachen Ausführung der Anordnung kann auf die T-Überwachung andererseits auch gänzlich verzichtet werden.

Das Gesamtsystem kann vollständig PC-gesteuert arbeiten, und die oben angesprochenen Steuersystemfunktionen können (auch unabhängig von einer PC-Konfiguration) weitgehend softwaremäßig realisiert sein.

## Patentansprüche

1. Ablationsanordnung (1) zur gezielten Erzeugung lokaler Läsionen in lebendem Gewebe im Körperinneren, insbesondere im Herzen, mit mindestens einer Energiequelle (15), einer auf einem Halteelement (11a) angebrachten Mehrzahl von Energieauskoppelelementen (11b bis 11g), einer Mehrzahl von die Energiequelle oder Energiequellen mit den Energieauskoppelelementen verbindenden Energieübertragungsleitungen (19.1) und den Energieübertragungsleitungen zugeordneten Schaltelementen (9.3) zur Herstellung oder Unterbrechung einer Verbindung zwischen der Energiequelle und jeweils einem Energieauskoppelelement, sowie mit Programmsteuermitteln (9.1; 100) zur zeitsequentiellen Ansteuerung mindestens eines Teils der Schaltelemente gemäß einem in einem Programmspeicher (100A) festgehaltenen Steuerprogramm, wobei die Energiequelle als HF-Leistungsquelle (15), das Halteelement als Katheterkörper (11a), die Energieauskoppelelemente als Elektroden (11b bis 11g), die Energieübertragungsleitungen als elektrische Leitungen (19.1) und die Schaltelemente als elektromechanische oder elektronische Schalter (9.3) ausgebildet sind und wobei die Energieauskoppelelemente (11b bis 11g) zugleich als erste Messfühler dienen und eine den Energieübertragungsleitungen (19.1) zuschaltbare erste Messsignal-Verarbeitungseinheit (107) zur Auswertung der aufgenommenen Messsignale vorgesehen ist, wobei die einzelnen Energieauskoppelelemente unter Steuerung durch die Programmsteuermittel (9.1; 100) dem Eingang der ersten Messsignal-Verarbeitungseinheit mindestens zeitweise zuschaltbar sind, wobei die Ablationsanordnung Mittel (107 bis 123) zur Veränderung eines gespeicherten Basis-Ablationsprogramms in Abhängigkeit von mindestens einer vor oder während der Behandlung eingegebenen und/oder einer während der Behandlung gemessenen Größe (IEGM, T) aufweist **dadurch gekennzeichnet, dass** die Elektroden (11b bis 11g) zur Aufnahme monophasischer Aktionspotentiale (MAP) als während der Behandlung gemessenen Größe und die erste Messsignal-Verarbeitungseinheit (107) zu deren Auswertung ausgebildet sind.

2. Ablationsanordnung nach Anspruch 1, **dadurch gekennzeichnet, dass** es sich bei dem Programmspeicher um einen Schreib-Lese-Speicher zum Aufnehmen von unterschiedlichen Programmen handelt.

3. Ablationsanordnung nach Anspruch 1 oder 2, **dadurch gekennzeichnet, dass** ein Stimulationsimpulsgenerator (5) vorgesehen ist, der in Abhängigkeit von einem Ausgangssignal der Programmsteuermittel (9.1; 100), insbesondere über mindestens eines der Schaltelemente (9.3) und mindestens eine Energieübertragungsleitung (19.1), mit einer der Elektroden (11b bis 11g) mindestens zeitweise verbindbar ist.

4. Ablationsanordnung nach einem der vorhergehenden Ansprüche, **dadurch gekennzeichnet, dass** die Mittel zur Beeinflussung des Ablationsprogramms eine Mehrzahl von den Energieauskoppelelementen (11b bis 11g) zugeordneten zweiten Messfühlern (17a bis 17f), insbesondere Temperaturfühlern, eine zweite Messsignal-Verarbeitungseinheit (9.9; 115) und mit dem Ausgang der zweiten Messsignal-Verarbeiungseinheit verbundene Schaltmittel (113) zur Veränderung des Ablationsprogramms in Abhängigkeit von mit den zweiten Messfühlern erfassten Messsignalen aufweisen.

5. Ablationsanordnung nach Anspruch 4, **gekennzeichnet durch** einen den zweiten Messfühlern (17a bis 17f) zugeordneten und **durch** die Programmsteuermittel (9.1; 100) in Abhängigkeit vom Ablationsprogramm gesteuerten Multiplexer (9.8) zur Aufnahme der Messfühlersignale über eine gegenüber der Anzahl der Messfühler verringerte Anzahl von Messsignal-Übertragungsleitungen oder -Anschlüssen (9.1e, 9.1f).

6. Ablationsanordnung nach einem der vorhergehenden Ansprüche, **dadurch gekennzeichnet, dass** eine einzelne Energiequelle (15) vorgesehen ist.

7. Ablationsanordnung nach einem der vorhergehenden Ansprüche, **dadurch gekennzeichnet, dass** die Programmsteuermittel (9.1; 100) mindestens einen Abschalt-Zeitgeber (9.5; 121) zur Sicherheitsabschaltung aller Energieübertragungsleitungen (19.1) bei Vorliegen einer vorbestimmten Abschaltbedingung aufweisen.

8. Ablationsanordnung nach einem der vorhergehenden Ansprüche, **dadurch gekennzeichnet, dass** die Programmsteuermittel (9.1) und die Schaltelemente (9.3) in einem separaten oder in das Basisgerät integrierten Schaltgerät (9) aufgenommen sind, das eingangsseitig über zwei serielle Datenleitungen, eine Stromversorgungsleitung und einen Energieübertragungsleitungs-Abschnitt mit einem Ablationsgerät (3) verbunden ist.

## Claims

1. An ablation arrangement (1) for the targeted production of local lesions in living tissue in the interior of a body, in particular in the heart, comprising at least one energy source (15), a plurality of energy coupling-out elements (11b to 11g), which is mounted on a holding element (11a), a plurality of energy transmission lines (19.1) connecting the energy source or sources to the energy coupling-out elements and switching elements (9.3) associated with the energy transmission lines for making or interrupting a connection between the energy source and a respective energy coupling-out element, and program control means (9.1; 100) for time-sequential actuation of at least a part of the switching elements in accordance with a control program stored in a program memory (100A), wherein the energy source is in the form of an HF power source (15), the holding element is in the form of a catheter body (11a), the energy coupling-out elements are in the form of electrodes (11b to 11g), the energy transmission lines are in the form of electrical lines (19.1) and the switching elements are in the form of electromechanical or electronic switches (9.3) and wherein the energy coupling-out elements (11b to 11g) serve at the same time as first measuring sensors and there is provided a first measuring signal processing unit (107) which can be connected to the energy transmission lines (19.1) for evaluation of the received measuring signals, wherein the individual energy coupling-out elements under the control of the program control means (9.1; 100) can be connected at least at times to the input of the first measuring signal processing unit, wherein the ablation arrangement has means (107 to 123) for changing a stored basic ablation program on dependence on at least one parameter (IEGM, T) which is input prior to or during the treatment and/or which is measured during the treatment, **characterised in that** the electrodes (11b to 11g) are adapted to record monophasic action potentials (MAP) as the parameter which is measured during the treatment and the first measuring signal processing unit (107) is adapted for evaluation thereof.

2. An ablation arrangement according to claim 1 **characterised in that** the program memory is a write-read memory for accommodating different programs.

3. An ablation arrangement according to claim 1 or claim 2 **characterised in that** there is provided a stimulation pulse generator (5) which can be connected at least at times to one of the electrodes (11b to 11g) in dependence on an output signal of the program control means (9.1; 100), in particular by way of at least one of the switching elements (9.3) and at least one energy transmission line (19.1).

4. An ablation arrangement according to one of the preceding claims **characterised in that** the means for influencing the ablation program have a plurality of second measuring sensors (17a to 17f) associated with the energy coupling-out elements (11b to 11g), in particular temperature sensors, a second measuring signal processing unit (9.9; 115) and switching means (113) connected to the output of the second measuring signal processing unit for varying the ablation program in dependence on measurement signals detected with the second measuring sensors.

5. An ablation arrangement according to claim 4 **characterised by** a multiplexer (9.8) which is associated with the second measuring sensors (17a to 17f) and which is controlled by the program control means (9.1; 100) in dependence on the ablation program for receiving the measuring sensor signals by way of a number of measuring signal transmission lines or connections (9.1e, 9.1f), which number is reduced in relation to the number of measuring sensors.

6. An ablation arrangement according to one of the preceding claims **characterised in that** there is provided a single energy source (15).

7. An ablation arrangement according to one of the preceding claims **characterised in that** the program control means (9.1; 100) have at least one switch-off timer (9.5; 121) for safety switch-off of all energy transmission lines (19.1) when a predetermined switch-off condition is present.

8. An ablation arrangement according to one of the preceding claims **characterised in that** the program control means (9.1) and the switching elements (9.3) are accommodated in a switching device (9) which is a separate device or which is integrated into the base device and which is connected at the input side to the ablation apparatus (3) by way of two serial data lines, a current supply line and an energy transmission line portion.

## Revendications

1. Dispositif d'ablation (1) pour générer, d'une manière ciblée, des lésions locales dans un tissu vivant à l'intérieur du corps, notamment dans le coeur, comportant une source d'énergie (15), une multiplicité d'éléments de découplage d'énergie (11b à 11g) disposés sur un élément de retenue (11a), une multiplicité de lignes de transmission d'énergie (19.1), reliant la source d'énergie ou les sources d'énergie aux éléments de découplage d'énergie, et d'éléments de commutation (9.3) associés aux lignes de transmission d'énergie, pour l'établissement ou l'interruption d'une liaison entre la source d'énergie et respectivement un élément de découplage d'énergie, ainsi que des moyens de commande à programme (9.1; 100) pour commander d'une manière séquentielle dans le temps au moins une partie des éléments de commutation conformément à un programme de commande qui est retenu de façon fixe dans une mémoire de programme (100A), et dans lequel la source d'énergie est agencée en tant que source de puissance HF (15), l'élément de retenue est agencé sous la forme d'un corps de cathéter (11a), les éléments de découplage d'énergie sont agencés sous la forme d'électrodes (11b, 11c), les lignes de transmission d'énergie sont agencées sous la forme de lignes électriques (19.1) et les éléments de commutation sont agencés sous la forme d'interrupteurs électromécaniques ou électroniques (9.3), et dans lequel les éléments de découplage d'énergie (11b à 11g) sont utilisés simultanément en tant que premiers capteurs de mesure, et il est prévu une première unité (107) de traitement de signaux de mesure, qui peut être connectée aux lignes de transmission d'énergie (19.1) et sert à évaluer les signaux de mesure enregistrés, et dans lequel les éléments individuels de découplage d'énergie peuvent être connectés au moins en partie sous la commande effectuée par des moyens de commande à programme (9.1; 100) à l'entrée de la première unité de traitement de signaux de mesure, et dans lequel le dispositif d'ablation comprend des moyens (107 à 123) pour modifier un programme d'ablation de base mémorisé, en fonction d'au moins une grandeur (IEGM,T) introduite avant ou pendant le traitement et/ou d'une grandeur (IEGM,T) mesurée pendant le traitement, **caractérisé en ce que** les électrodes (11b à 11g) sont agencées pour enregistrer des potentiels d'action monophasés (MAP) en tant que grandeurs mesurées pendant le traitement et que la première unité (107) de traitement des signaux de mesure est agencée pour leur évaluation.

2. Dispositif d'ablation selon la revendication 1, **caractérisé en ce qu'**en ce qui concerne la mémoire de programme, il s'agit d'une mémoire d'écriture et de lecture pour l'enregistrement de programmes différents.

3. Dispositif d'ablation selon la revendication 1 ou 2, **caractérisé en ce qu'**il est prévu un générateur d'impulsions de stimulation (5), qui peut être relié au moins par instants à l'une des électrodes (11b à 11g) en fonction d'un signal de sortie des moyens de commande à programme (9.1; 100), notamment par l'intermédiaire d'au moins l'un des éléments de commutation (9.3) et d'au moins une ligne de transmission d'énergie (19.1).

4. Dispositif d'ablation selon l'une des revendications précédentes, **caractérisé en ce que** les moyens pour influer sur le programme d'ablation comportent une multiplicité de seconds capteurs de mesure (17a à 17f), notamment des capteurs de température, associés aux éléments de découplage d'énergie (11b à 11g), une seconde unité de traitement (9.9; 115) de traitement de signaux de mesure et des moyens de commutation (113), reliés à la sortie de la seconde unité de traitement de signaux de mesure pour modifier le programme d'ablation en fonction des signaux de mesure détectés par les seconds capteurs de mesure.

5. Dispositif d'ablation selon la revendication 4, **caractérisé par** un multiplexeur (9.8) qui est associé aux seconds capteurs de mesure (17a à 17f) et sont commandés par les moyens de commande à programme (9.1; 100) en fonction du programme d'ablation, pour l'enregistrement des signaux des capteurs de mesure par l'intermédiaire d'un nombre de lignes ou de bornes de transmission de signaux (9.1e, 9.1f), qui est réduit par rapport au nombre des capteurs de mesure.

6. Dispositif d'ablation selon l'une des revendications précédentes, **caractérisé en ce qu'**il est prévu une source individuelle d'énergie (15).

7. Dispositif d'ablation selon l'une des revendications précédentes, **caractérisé en ce que** les moyens de commande à programme (9.1; 100) possèdent au moins une minuterie de débranchement (9.5; 121) pour réaliser un débranchement de sécurité de toutes les lignes de transmission d'énergie (19.1) dans le cas de la présence d'une condition prédéterminée de débranchement.

8. Dispositif d'ablation selon l'une des revendications précédentes, **caractérisé en ce que** les moyens de commande à programme (9.1) et les éléments de commutation (9.3) sont enregistrés dans un appareil de commutation (9) séparé ou intégré dans l'appareil de base et qui est relié d'un côté à un appareil d'ablation (3) au moyen de deux lignes de transmission de données en série, d'une ligne d'alimentation en courant et d'une section de ligne de transmission d'énergie.
